# EUROPÄISCHE PATENTANMELDUNG

(11) **EP 4 033 238 A1**
(43) Veröffentlichungstag der Anmeldung: **27.07.2022**
(21) Anmeldenummer: 22154342.4
(22) Anmeldetag: 02.10.2017
(51) Int. Cl.: G01N 29/024, G01N 33/18

(54) **FLÜSSIGKEITSRÜCKHALTESYSTEM MIT EINEM ULTRASCHALLSENSOR**

(30) Priorität: 02.10.2016 DE 202016105482 U
(62) Teilanmeldung aus: 17194370.7
(71) Anmelder: IT Inventor GmbH, 48465 Samern (DE)
(72) Erfinder: Satar, Mehmet, 48531 Nordhorn (DE); Thannhäuser, Ingo, 48465 Samern (DE)
(74) Vertreter: Werner & ten Brink

(57) **Zusammenfassung**

Die Erfindung ist ein Flüssigkeitsrückhaltesystem (12) mit zumindest einer unter einem Aggregat (20) einer leckagegefährdeten Anlage platzierten Auffangwanne (14) und einem Ultraschallsensor (10), wobei mittels des Ultraschallsensors (10) ein Sensorsignal (26) generierbar ist, welches eine Erkennung von Glykol in einem Wasser-Glykol-Gemisch (22), dem der Ultraschallsensor (10) ausgesetzt ist, kodiert und wobei mittelbar oder unmittelbar aufgrund des Sensorsignals (26) ein stromabwärts der Auffangwanne (14) platziertes Ventil (18) ansteuerbar ist.

## Beschreibung

Die Erfindung betrifft ein Flüssigkeitsrückhaltesystem mit einem zur Sensierung von ausgetretenem Glykol oder anderen insbesondere wasserlöslichen Stoffen in einer mittels der Auffangvorrichtung aufgefangenen Flüssigkeit bestimmten Ultraschallsensor.

Glykol (umgangssprachliche Bezeichnung für (Mono-) Ethylenglykol) wird bekanntlich als Frostschutzmittel, zum Beispiel in Klima- oder Kälteanlagen, eingesetzt. Im Falle einer Leckage solcher Anlagen droht ein Austritt des Frostschutzmittels. Weil Glykol als wassergefährdender Stoff eingestuft und überdies gesundheitsschädlich ist, muss bei einer Leckage speziell ein Versickern des Frostschutzmittels oder ein sonstiger Übergang des Frostschutzmittels in die Umwelt verhindert werden.

Weil Glykol sehr gut wasserlöslich ist, lässt sich Glykol - anders als zum Beispiel hydrophobe Stoffe wie Öl und dergleichen - mit einem Flüssigkeitsabscheider oder einer Rückhaltevorrichtung, die auf einer Phasenbildung der abzuscheidenden/zurückzuhaltenden Flüssigkeit einerseits und Wasser andererseits basiert, nicht abscheiden/zurückhalten. Die bisherigen Lösungen zur Vermeidung eines Übertritts von Glykol in die Umwelt basieren daher auf der Verwendung eines Flüssigkeitsrückhaltesystems mit zumindest einer Auffangwanne, die unter einer jeweiligen leckagegefährdeten Anlage aufgebaut wird oder in der die jeweilige leckagegefährdete Anlage platziert wird. Die nachfolgende Beschreibung wird im Interesse einer besseren Lesbarkeit, aber ohne Verzicht auf eine weitergehende Allgemeingültigkeit, am Beispiel eines Flüssigkeitsrückhaltesystems mit genau einer Auffangwanne fortgesetzt. Ein Flüssigkeitsrückhaltesystem mit einer Mehrzahl von Auffangwannen ist dabei stets mitzulesen.

Eine Auffangwanne ist bisher stets so dimensioniert, dass eine bestimmte Menge Regenwasser zusätzlich zu eventuell ausgetretenem Glykol aufgenommen werden kann, ohne dass es zu einem Überlauf kommt. Die Dimensionierung einer Auffangwanne ergibt sich dabei speziell aus am Aufstellungsort in der Vergangenheit beobachteten maximalen Regenmengen einerseits und einem realistischen Wartungs-/Prüfzyklus andererseits. Bei einer Leckage sind eine Entleerung der Auffangwanne und eine anschließende sachgerechte Entsorgung des in der Auffangwanne aufgefangenen Wasser-Glykol-Gemisches erforderlich.

Zur Vermeidung starrer Wartungs-/Prüfzyklen, die eine optische Inspektion der Auffangwanne(n) durch entsprechend geschultes Personal erfordern, kann eine Drucküberwachung der jeweiligen Anlage vorgesehen sein, wobei ein Abfallen eines in der jeweiligen Anlage gemessenen Druckmesswerts unter einen vorgegebenen Grenzwert auf eine Leckage der Anlage hindeutet und daraus ein Alarmsignal für Wartungspersonal generiert wird, das daraufhin die Auffangwanne(n) prüft und gegebenenfalls für eine Entsorgung eines dort aufgefangenen Wasser-Glykol-Gemisches sorgt.

Ein solches Vorgehen ist aufwändig und zudem fehleranfällig, wenn nämlich das Alarmsignal nicht beachtet oder nicht wahrgenommen wird oder wenn das Alarmsignal zusammen mit anderen Alarmsignalen eintrifft und das Wartungspersonal entsprechend eine Entscheidung treffen muss, welcher Alarmmeldung zunächst nachgegangen wird, oder das Wartungspersonal sogar aufgrund einer übergeordneten Alarmmeldung veranlasst wird, ein jeweiliges Betriebsgelände zu verlassen.

Zur Vermeidung solcher Nachteile wird in der DE 20 2012 103 850 U die Verwendung einer Sensorik zum Erkennen von Glykol in einer Flüssigkeit und damit zum Erkennen eines Glykolaustritts aus einer Anlage der oben genannten Art vorgeschlagen. Wenn ein Glykolaustritt sensiert wird, wird mittels der Sensorik ein entsprechendes Signal erzeugt und auf Basis des Signals ein einem Auslass der Auffangwanne zugeordnetes Ventil angesteuert, so dass ein Abfluss von Glykol aus der Auffangwanne verhindert wird.

Eine Aufgabe der vorliegenden Erfindung besteht darin, eine verbesserte Ausführungsform eines einen Ultraschallsensor umfassenden Flüssigkeitsrückhaltesystems anzugeben.

Diese Aufgabe wird mittels eines Flüssigkeitsrückhaltesystems mit den Merkmalen des Anspruchs 1 gelöst. Eine solches Flüssigkeitsrückhaltesystem umfasst in grundsätzlich an sich bekannter Art und Weise (siehe zum Beispiel die DE 20 2012 103 850 U) einen Ultraschallsensor, zum Beispiel einen als Glykolsensor fungierenden Ultraschallsensor, sowie zumindest eine unter einem Aggregat einer leckagegefährdeten Anlage platzierte Auffangwanne. Mittels des Ultraschallsensors ist ein Sensorsignal generierbar, welches eine Erkennung von zum Beispiel Glykol in einem Wasser-Glykol-Gemisch, dem der Ultraschallsensor ausgesetzt ist, kodiert. Mittelbar oder unmittelbar aufgrund des Sensorsignals ist ein stromabwärts der Auffangwanne platziertes Ventil ansteuerbar. Das Flüssigkeitsrückhaltesystem zeichnet sich dadurch aus, dass der Ultraschallsensor stromabwärts der Auffangwanne in einer Sensoraufnahmevorrichtung platziert ist. Die Sensoraufnahmevorrichtung umfasst einen in Relation zu einem Bodenniveau der Auffangwanne tiefer liegenden Bodenabschnitt, in welchem sich zumindest ein Teil eines aus der Auffangwanne abfließenden Fluids sammelt ("Reservoir"). Aus der Auffangwanne fließt im Falle eines Glykolaustritts aus der leckagegefährdeten Anlage ein Wasser-Glykol-Gemisch ab. Ohne einen Fehlerfall fließt aus der Auffangwanne nicht verunreinigtes Wasser, zum Beispiel aufgefangenes Regenwasser, ab. Deshalb wird allgemein der Ausdruck Fluid verwendet, wenn es sich um ein Wasser-Glykol-Gemisch oder nicht verunreinigtes Wasser handelt. Der Ultraschallsensor weist an einer insbesondere gabelförmigen Halterung einen Ultraschall-Sender und einen Ultraschall-Empfänger auf. Der Ultraschallsensor ist von der Sensoraufnahmevorrichtung in einer Art und Weise aufgenommen, dass der Ultraschall-Sender und der Ultraschall-Empfänger in den Bereich des tiefer liegenden Bodenabschnitts reichen und bei einem aus der Auffangwanne ausströmenden Fluid zumindest im Wesentlichen von dem strömenden Fluid oder zumindest dem im Bodenabschnitt zurückgehaltenen Fluid benetzt werden.

Der Vorteil eines solchen Flüssigkeitsrückhaltesystems besteht darin, dass auch bei einem nur in einer geringen Menge aus der Auffangwanne abfließenden Fluid der Ultraschall-Sender und der Ultraschall-Empfänger des Ultraschallsensors zumindest in einem Umfang mit dem Fluid in Kontakt kommen, dass ein verlässliches Sensorsignal generierbar ist, nämlich ein Sensorsignal, welches verlässlich zum Beispiel einen eventuellen Glykolaustritt aus der leckagegefährdeten Anlage anzeigt.

Vorteilhafte Ausgestaltungen der Erfindung sind Gegenstand der Unteransprüche. Dabei verwendete Rückbeziehungen weisen auf die weitere Ausbildung des Gegenstandes des Hauptanspruches durch die Merkmale des jeweiligen Unteranspruches hin. Sie sind nicht als ein Verzicht auf die Erzielung eines selbständigen, gegenständlichen Schutzes für die Merkmalskombinationen der rückbezogenen Unteransprüche zu verstehen. Des Weiteren ist im Hinblick auf eine Auslegung der Ansprüche sowie der Beschreibung bei einer näheren Konkretisierung eines Merkmals in einem nachgeordneten Anspruch davon auszugehen, dass eine derartige Beschränkung in den jeweils vorangehenden Ansprüchen sowie bei einer allgemeineren Ausführungsform des gegenständlichen Ultraschallsensors nicht vorhanden ist. Jede Bezugnahme in der Beschreibung auf Aspekte nachgeordneter Ansprüche ist demnach ausdrücklich als Beschreibung optionaler Merkmale zu lesen.

Bei einer Ausführungsform des Flüssigkeitsrückhaltesystems umfasst die Sensoraufnahmevorrichtung eine von einer Hüllfläche der Sensoraufnahmevorrichtung zurückgesetzte Einstülpung zur Aufnahme des Ultraschallsensors. Der Ultraschallsensor ist in der Sensoraufnahmevorrichtung am Boden der Einstülpung fixiert. Eine zumindest den Ultraschall-Sender und den Ultraschall-Empfänger umfassende Sensorik des Ultraschallsensors schließt im Innern der Sensoraufnahmevorrichtung an eine Innenseite des Bodens der Einstülpung an und eine Steuerungseinheit des Ultraschallsensors befindet sich auf einer der Innenseite des Bodens der Einstülpung gegenüberliegenden Seite. Die den Ultraschallsensor aufnehmende Einstülpung fungiert als mechanischer Schutz des Ultraschallsensors. Alle Funktionseinheiten zur Sensierung von zum Beispiel Glykol in einem aus der Auffangwanne des Flüssigkeitsrückhaltesystems austretenden Fluid sind im Bereich der Sensoraufnahmevorrichtung konzentriert. Der Ultraschallsensor ist dafür gewissermaßen in zwei Abschnitte unterteilt. Ein Abschnitt mit den sensorisch wirksamen Teilen des Ultraschallsensors befindet sich im Innern der Sensoraufnahmevorrichtung und entsprechend auf der Innenseite des Bodens der Einstülpung. Ein Abschnitt mit einer Steuerungseinheit des Ultraschallsensors befindet sich auf einer gegenüberliegenden Seite des Bodens der Einstülpung. Dieser Abschnitt kommt nicht mit einem aus der Auffangwanne abfließenden und durch die Sensoraufnahmevorrichtung fließenden Fluid in Kontakt. An diesen Abschnitt ist entsprechend ein Anschlusskabel des Ultraschallsensors zur Energieversorgung sowie zur Ableitung zumindest eines mittels des Ultraschallsensors im Betrieb generierbaren Sensorsignals angeschlossen.

Bei einer besonderen Ausführungsform des gegenständlichen Flüssigkeitsrückhaltesystems und dessen Sensoraufnahmevorrichtung befindet sich die Sensorik des Ultraschallsensors unmittelbar im Anschluss an den Boden der Einstülpung im Innern der Sensoraufnahmevorrichtung und die Steuerungseinheit befindet sich ebenfalls unmittelbar im Anschluss an den Boden der Einstülpung auf der gegenüberliegenden Seite des Bodens der Einstülpung . Die Sensorik und die Steuerungseinheit sind damit nur minimal voneinander beabstandet und es ergibt sich entsprechend eine kompakte Bauform des Ultraschallsensors und der Sensoraufnahmevorrichtung insgesamt.

Bei einer weiteren besonderen Ausführungsform des gegenständlichen Flüssigkeitsrückhaltesystems und dessen Ultraschallsensors erzeugt der Ultraschallsensor als Sensorsignal ein Binärsignal. Das Binärsignal kodiert mit einem ersten Status, dass kein Glykol oder nur eine Glykolkonzentration unterhalb eines vorgegebenen oder vorgebbaren Schwellwerts sensiert wurde, und mit einem komplementären zweiten Status, dass Glykol oder eine Glykolkonzentration oberhalb des Schwellwerts sensiert wurde. Ein solches Binärsignal kann von einer nachfolgenden Funktionseinheit unmittelbar verarbeitet werden. Zum Beispiel kann das Binärsignal unmittelbar als Steuersignal an das Ventil des Flüssigkeitsrückhaltesystems weitergeleitet werden, wo abhängig vom Status des Binärsignals ein Öffnen oder ein Schließen des Ventils resultiert. Dies ist eine unmittelbare Ansteuerung des Ventils aufgrund des Sensorsignals. Auch bei einer mittelbaren Ansteuerung des Ventils aufgrund des Sensorsignals, etwa mittels einer zentralen Verarbeitungseinheit, welche das Sensorsignal empfängt und aufgrund des Sensorsignals ein Steuersignal für das Ventil generiert, ist auf Seiten der Verarbeitungseinheit keine spezielle Verarbeitung des Sensorsignals erforderlich und das Steuersignal kann ohne Zeitverzug generiert werden. - So wie hier und im Folgenden die Begriffe "Glykol" und "Glykolkonzentration" im Zusammenhang mit einer Sensierung eines zu sensierenden Stoffs oder einer Sensierung einer Konzentration des zu sensierenden Stoffs genannt sind, gilt, dass diese Begriffe auch stellvertretend für andere zu sensierende Stoffe und deren Konzentration stehen.

Bei einer bevorzugten Ausführungsform des gegenständlichen Flüssigkeitsrückhaltesystems und dessen Ultraschallsensors ist vorgesehen, dass der Ultraschallsensor einen Sender und einen Empfänger zum Aussenden bzw. Empfangen von Ultraschallsignalen oder allgemein von Schallwellen aufweist und dass der Ultraschallsensor des Weiteren eine Steuerungseinheit zum Aktivieren des Senders und zum Erkennen des Empfangs eines ausgesandten Ultraschallsignals beim Empfänger aufweist. Hinsichtlich der Funktionalität der Steuerungseinheit zeichnet sich der Ultraschallsensor dadurch aus, dass mittels der Steuerungseinheit ein Maß für eine Laufzeit eines Ultraschallsignals durch die jeweils zu prüfende Flüssigkeit vom Sender zum Empfänger ermittelbar ist und dass mittels der Steuerungseinheit anhand zweier Parameter, nämlich einerseits anhand des ermittelten Maßes für die Laufzeit des Ultraschallsignals und andererseits anhand eines Temperaturmesswerts, eine Glykolkonzentration in dem Fluid, dem der Ultraschallsensor ausgesetzt ist, also zum Beispiel einem Wasser-Glykol-Gemisch, ermittelbar ist.

Durch die Berücksichtigung nicht nur des ermittelten Maßes für die Laufzeit des Ultraschallsignals, sondern auch eines Temperaturmesswerts, ist der Ultraschallsensor ein Ultraschallsensor mit Temperaturkompensation. Die Laufzeitmessung liefert ein Maß für die jeweilige Dichte der geprüften Flüssigkeit, also zum Beispiel des Wasser-Glykol-Gemisches. Die Dichte des Gemisches steigt mit der im Gemisch enthaltenen Glykolmenge. Die Dichte des Gemisches ist aber auch temperaturabhängig. Der Vorteil eines Ultraschallsensors mit Temperaturkompensation besteht in der automatischen Anpassung zum Beispiel an eine Umgebungstemperatur oder eine Temperatur des Wasser-Glykol-Gemisches. Das Erkennungsergebnis eines solchen Ultraschallsensors ist gegenüber einem Ultraschallsensor ohne Temperaturkompensation verbessert. Mittels der Temperaturkompensation ist demgemäß gewährleistet, dass die Erkennung eines eventuellen Glykolaustritts unabhängig von der jeweiligen Umgebungstemperatur oder der Temperatur des Wasser-Glykol-Gemisches so genau sowie so früh und so schnell wie möglich erfolgt.

Grundsätzlich kommt ein Flüssigkeitsrückhaltesystem mit einem Ultraschallsensor mit Temperaturkompensation auch ohne eine Sensoraufnahmevorrichtung mit einem in Relation zu einem Bodenniveau der Auffangwanne tiefer liegenden Bodenabschnitt in Betracht. Ein solches Flüssigkeitsrückhaltesystem zeichnet sich neben den Merkmalen des Oberbegriffs des Anspruchs 1 dadurch aus, dass dessen Ultraschallsensor - wie oben beschrieben - einen Ultraschall-Sender und einem Ultraschall-Empfänger sowie eine Steuerungseinheit umfasst, dass mittels der Steuerungseinheit ein Maß für eine Laufzeit eines Ultraschallsignals vom Ultraschall-Sender zum Ultraschall-Empfänger ermittelbar ist und dass mittels der Steuerungseinheit anhand des ermittelten Maßes für die Laufzeit des Ultraschallsignals und anhand eines Temperaturmesswerts zum Beispiel eine Glykolkonzentration in einem Wasser-Glykol-Gemisch ermittelbar ist, dem der Ultraschallsensor ausgesetzt ist. Der Ultraschallsensor kann stromaufwärts der Auffangwanne, zum Beispiel in der Auffangwanne, oder stromabwärts der Auffangwanne, zum Beispiel in einer von der Auffangwanne kommenden Rohrleitung, angeordnet sein. Die im Folgenden beschriebenen optionalen Ausführungsformen eines Ultraschallsensors mit Temperaturkompensation können auch ein solches Flüssigkeitsrückhaltesystem optional ergänzen.

Für die Automobiltechnik ist aus der DE 10 2005 043 699 A1 eine einen Ultraschall-Sender und einen Ultraschall-Empfänger umfassende Sensoreinheit für ein Fahrzeug bekannt, mittels derer ein Korrosionsschutzmittelanteil in einem Fluid eines Fluidsystems ermittelbar ist. Die Sensoreinheit ermittelt neben einer Laufzeit des Ultraschallsignals durch das Fluid auch die Temperatur des Fluids und berücksichtigt die Temperatur bei der Ermittlung des Korrosionsschutzmittelanteils.

Bei einer speziellen Ausführungsform des Ultraschallsensors sind dessen Sender und Empfänger einander gegenüberliegend angeordnet, insbesondere einander koaxial gegenüberliegend angeordnet. Die Laufzeit wird dann für die Zeit ermittelt, welche das Ultraschallsignal auf direktem Weg vom Sender zum Empfänger benötigt. Eine Alternative zu dieser Ausführungsform besteht darin, dass Sender und Empfänger auf derselben Seite einer Reflexionsfläche angeordnet sind. Dies führt zu einer verdoppelten Weglänge für das Ultraschallsignal (vom Sender zur Reflexionsfläche und von der Reflexionsfläche zurück zum Empfänger).

Bei einer weiteren speziellen Ausführungsform des Ultraschallsensors umfasst dieser selbst einen Temperatursensor und mittels der Steuerungseinheit des Ultraschallsensors ist anhand eines vom Temperatursensor erhältlichen Temperatursignals ein Temperaturmesswert ermittelbar. Indem der Ultraschallsensor den Temperatursensor selbst umfasst, ist die gesamte notwendige Sensorik im Ultraschallsensor zusammengefasst. Alternativ kann der Temperatursensor auch unabhängig vom Ultraschallsensor und beabstandet vom Ultraschallsensor platziert sein. Dann sind der Temperatursensor und der Ultraschallsensor geeignet kommunikativ verbunden, so dass dem Ultraschallsensor ein Temperatursignal des Temperatursensors übermittelbar ist und beim Betrieb übermittelt wird.

Bei einer bevorzugten Ausführungsform des Flüssigkeitsrückhaltesystems und des davon umfassten Ultraschallsensors ist zum Beispiel eine Glykolkonzentration in einem Wasser-Glykol-Gemisch, dem der Ultraschallsensor ausgesetzt ist, mittels der Steuerungseinheit mittels einer Umsetzungstabelle und darin gespeicherter, empirisch ermittelter Werte ermittelbar. Die Umsetzungstabelle umfasst dafür empirisch ermittelte Daten für verschiedene Temperaturen und verschiedene Glykolkonzentrationen und der Zugriff auf die Umsetzungstabelle erfolgt mittels des Temperaturmesswerts und anschließend mittels des ermittelten Maßes für die Laufzeit des Ultraschallsignals (oder umgekehrt). Anhand der Umsetzungstabelle resultiert eine ermittelte, zu dem Temperaturmesswert und dem Maß für die Laufzeit des Ultraschallsignals gehörige Glykolkonzentration. Diese kann als Messwert ausgegeben werden oder an eine nachgeordnete Einheit, zum Beispiel eine Anzeigeeinheit oder eine Alarmeinheit, übermittelt werden. Alternativ oder zusätzlich kann mittels des Ultraschallsensors anhand der jeweils ermittelten Glykolkonzentration sowie eines bezüglich einer tolerierbaren Glykolkonzentration vorgegebenen oder vorgebbaren Schwellwerts überprüft werden, ob die ermittelte Glykolkonzentration den Schwellwert überschreitet. Im Falle einer Schwellwertüberschreitung ist dann mittels des Ultraschallsensors automatisch ein Sensorsignal generierbar, das zum Beispiel zur Ansteuerung eines Ventils zum Verschließen eines Abflusses einer Auffangwanne oder zur Ansteuerung eines sonstigen Aktors verwendbar ist.

Bei einer nochmals weiteren Ausführungsform umfasst der Ultraschallsensor eine Busschnittstelle, zum Beispiel eine CAN-Bus-Schnittstelle. Eine Busschnittstelle erlaubt eine einfache und schnelle Datenübertragung vom und zum Ultraschallsensor, zum Beispiel zur Steuerungseinheit, und auf diesem Wege können der Steuerungseinheit zum Beispiel die Daten für die Umsetzungstabelle und/oder ein bei der Generierung des Binärsignals zu berücksichtigender Schwellwert eingeprägt werden.

Die Erfindung ist schließlich auch ein Flüssigkeitsrückhaltesystem, insbesondere ein Flüssigkeitsrückhaltesystem mit einer Auffangwanne, in welcher sich in einem Schadensfall zum Beispiel ein Wasser-Glykol-Gemisch sammelt, mit einem Ultraschallsensor der hier und im Folgenden beschriebenen Art oder mit einem Ultraschallsensor und einer Sensoraufnahmevorrichtung der hier und im Folgenden beschriebenen Art. Der Ultraschallsensor ist dem Flüssigkeitsrückhaltesystem in geeigneter Art und Weise zugeordnet. Beispielsweise ist der Ultraschallsensor in der Auffangwanne in einer Art und Weise platziert ist, dass er mit einem dort gegebenenfalls vorliegenden Wasser-Glykol-Gemisch in Kontakt kommt. Alternativ ist der Ultraschallsensor mit der Sensoraufnahmevorrichtung stromabwärts der Auffangwanne angeordnet und kommt dort mit dem aus der Auffangwanne abfließenden Fluid und somit gegebenenfalls ebenfalls mit einem Wasser-Glykol-Gemisch in Kontakt. Der Ultraschallsensor ermittelt zum Beispiel eine Glykolkonzentration in dem Wasser-Glykol-Gemisch. Der ermittelte Wert für die Glykolkonzentration ist aufgrund der Berücksichtigung der Temperatur besonders genau. Wenn die ermittelte Glykolkonzentration über einem vorgegebenen oder vorgebbaren Schwellwert liegt, erzeugt der Ultraschallsensor ein Sensorsignal, mittels dessen die Überschreitung des Schwellwerts angezeigt wird. Mittels des Sensorsignals kann zum Beispiel eine Alarmeinrichtung, also zum Beispiel eine optische oder akustische Alarmeinrichtung, aktiviert werden, um auf diese Weise auf den Schadensfall aufmerksam zu machen. Zusätzlich oder alternativ kann auch ein Aktor des Flüssigkeitsrückhaltesystems, zum Beispiel ein einem Abfluss der Auffangwanne zugeordnetes oder allgemein ein stromabwärts der Auffangwanne befindliches Ventil, angesteuert werden. Bei einer Ansteuerung eines Ventils oder dergleichen aufgrund des Sensorsignals bewirkt das Sensorsignal ein Schließen des Ventils, so dass ein Abfluss des Wasser-Glykol-Gemisches aus der Auffangwanne verhindert wird.

Alternativ kommt die Verwendung des Ultraschallsensors oder der Sensoraufnahmevorrichtung mit einem Ultraschallsensor zum Beispiel auch im Rahmen einer Dachentwässerung, zum Beispiel bei einem Dach mit einer darauf platzierten Solaranlage, in Betracht. Der Ultraschallsensor ist dann zum Beispiel in einer Dachrinne, einem Fallrohr oder einer sonst geeigneten Stelle angebracht. Ein mittels eines solchen Ultraschallsensors generierbares Sensorsignal kann zum Beispiel zur Ansteuerung eines Ventils zum Absperren des Fallrohrs oder eines Rohrs in einem weiterleitenden Rohrsystem oder zur Ansteuerung eines Ventils oder einer Gruppe von Ventilen in einem Rohrsystem zur Umleitung der vom jeweiligen Dach kommenden Flüssigkeiten verwendet werden. Insoweit ist die Erfindung auch eine Vorrichtung zur Dachentwässerung, insbesondere zur Dachentwässerung eines eine Solaranlage tragenden Dachs, mit einer Dachrinne und/oder einem Fallrohr sowie zumindest einem in der Dachrinne, in dem Fallrohr und/oder auf der Dachfläche platzierten Ultraschallsensor.

Schließlich kommt die Verwendung des Ultraschallsensors oder der Sensoraufnahmevorrichtung mit einem Ultraschallsensor zum Beispiel auch allgemein in einem Rohrsystem in Betracht, wobei mittels des Ultraschallsensors eine Glykolkonzentration oder dergleichen des durch das Rohrsystem strömenden Mediums ermittelt und ggf. ein Sensorsignal erzeugt wird und wobei das Sensorsignal zur Ansteuerung eines Ventils oder einer Gruppe von Ventilen zur Absperrung einer Strecke in dem Rohrsystem oder zur Umleitung des durch das Rohrsystem strömenden Mediums verwendet wird.

Die Verwendung des hier und im Folgenden vorgeschlagenen Ultraschallsensors, der während der Messung mit der zu prüfenden Flüssigkeit in Kontakt ist, kommt auch zusammen mit zumindest einem weiteren Glykolsensor, der während der Messung nicht mit der zu prüfenden Flüssigkeit in Kontakt ist, zum Beispiel einem Glykolsensor gemäß der DE 20 2012 103 850 U, in Betracht. Eine solche Kombination von zwei oder ggf. auch mehr als zwei Sensoren kann zum Beispiel in der Form betrieben werden, dass eine Sensierung von Glykol durch einen der beiden Sensoren mittels des anderen Sensors gewissermaßen überprüft wird und ein Sensorsignal zum Ansteuern eines Aktors, zum Beispiel eines Ventils oder dergleichen, erst ausgelöst wird, wenn sowohl der erste Sensor wie auch der zweite Sensor eine Sensierung von Glykol oberhalb eines jeweiligen Schwellwerts melden. Alternativ kommt auch in Betracht, einen Mittelwert oder ein sonstiges gewichtetes Mittel der Messwerte einer Gruppe von Sensoren, von denen zumindest einer mit der zu prüfenden Flüssigkeit in Kontakt ist und zumindest einer mit der zu prüfenden Flüssigkeit nicht in Kontakt ist, zu verwenden und eine Ansteuerung eines Aktors, zum Beispiel eines Ventils oder dergleichen, automatisch dann auszulösen, wenn der jeweils ermittelte Wert einen vorgegebenen oder vorgebbaren Schwellwert überschreitet.

Die mit der Anmeldung eingereichten Ansprüche sind Formulierungsvorschläge ohne Präjudiz für die Erzielung weitergehenden Schutzes. Da speziell die Gegenstände der Unteransprüche im Hinblick auf den Stand der Technik am Prioritätstag eigene und unabhängige Erfindungen bilden können, behält die Anmelderin sich vor, diese oder noch weitere, bisher nur in der Beschreibung und/oder Zeichnung offenbarte Merkmalskombinationen zum Gegenstand unabhängiger Ansprüche oder Teilungserklärungen zu machen. Sie können weiterhin auch selbständige Erfindungen enthalten, die eine von den Gegenständen der vorhergehenden Unteransprüche unabhängige Gestaltung aufweisen.

Nachfolgend wird ein Ausführungsbeispiel der Erfindung anhand der Zeichnung näher erläutert. Einander entsprechende Gegenstände oder Elemente sind in allen Figuren mit den gleichen Bezugszeichen versehen. Das Ausführungsbeispiel ist nicht als Einschränkung der Erfindung zu verstehen. Vielmehr sind im Rahmen der vorliegenden Offenbarung auch Abänderungen und Modifikationen möglich, insbesondere solche Varianten, die zum Beispiel durch Kombination oder Abwandlung von einzelnen in Verbindung mit den im allgemeinen oder speziellen Beschreibungsteil beschriebenen sowie in den Ansprüchen und/oder der Zeichnung enthaltenen Merkmalen bzw. Elementen oder Verfahrensschritten für den Fachmann im Hinblick auf die Lösung der Aufgabe entnehmbar sind und durch kombinierbare Merkmale zu einem neuen Gegenstand oder zu neuen Verfahrensschritten bzw. Verfahrensschrittfolgen führen.

Es zeigen
- Fig. 1: ein Flüssigkeitsrückhaltesystem mit einem zum Beispiel als Glykolsensor fungierenden Ultraschallsensor,
- Fig. 2: einen Glykolsensor,
- Fig. 3: den Glykolsensor gemäß Fig. 2 in einer Sensoraufnahmevorrichtung,
- Fig. 4: eine weitere Ausführungsform einer Sensoraufnahmevorrichtung zur Aufnahme eines Glykolsensors gemäß Fig. 2,
- Fig. 5: ein Flüssigkeitsrückhaltesystem mit einer Sensoraufnahmevorrichtung gemäß Fig. 4 und einem davon aufgenommenen Glykolsensor,
- Fig. 6: wesentliche Funktionseinheiten des Glykolsensors gemäß Fig. 2,
- Fig. 7: eine Kurvenschar für resultierende Schallgeschwindigkeiten in einem Wasser-Glykol-Gemisch bei unterschiedlichen Glykolkonzentrationen und unterschiedlichen Temperaturen sowie
- Fig. 8: eine zur Verwendung in einem Glykolsensor gemäß Fig. 2 vorgesehene Umsetzungstabelle mit den Daten der Kurvenschar gemäß Fig. 7.

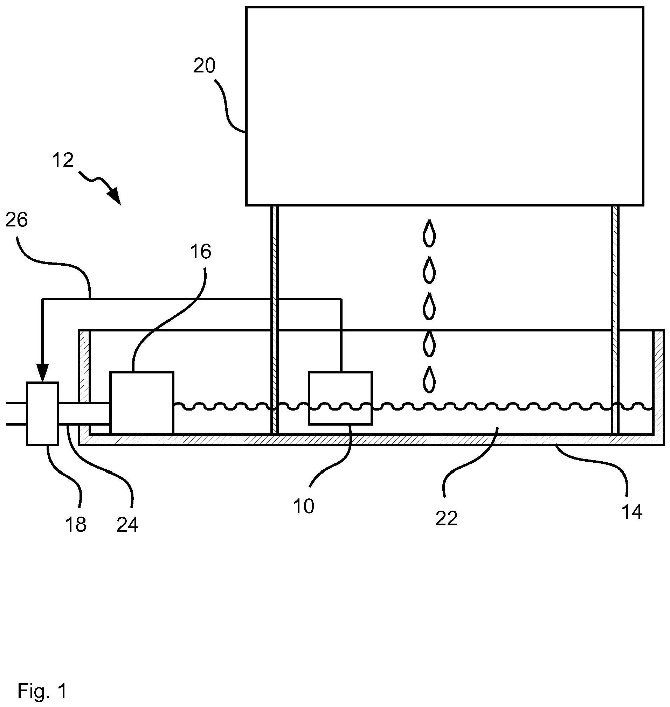

Die Darstellung in Figur 1 zeigt schematisch vereinfacht zur Erläuterung eines Anwendungsbeispiels für einen Glykolsensor 10 in einer teilweise geschnittenen Seitenansicht ein Flüssigkeitsrückhaltesystem 12. Dieses umfasst eine Auffangwanne 14, einen grundsätzlich optionalen Abscheider 16, zum Beispiel einen Abscheider 16 in Form einer Flüssigkeitsrückhaltevorrichtung 16 gemäß der EP 2 335 794 B oder der EP 2 716 827 A, und ein Ventil 18 stromabwärts der Flüssigkeitsrückhaltevorrichtung 16. Bei dem Glykolsensor 10 handelt es sich um einen hinsichtlich des zugrunde liegenden Messprinzips grundsätzlich an sich bekannten Ultraschallsensor.

Die Auffangwanne 14 befindet sich unter einer Klimaanlage oder dergleichen, die hier als Beispiel für ein Aggregat 20 genannt ist, aus dem im Falle einer Leckage ein flüssiges, wassergefährdendes Medium, zum Beispiel Glykol, austritt, wie dies in der Darstellung in Figur 1 durch die Tropfenlinie angedeutet ist. Mittels des Glykolsensors 10 soll beim Betrieb des Flüssigkeitsrückhaltesystems 12 ein Austritt eines wassergefährdenden Mediums, insbesondere Glykol, erkannt werden.

Im Interesse einer besseren Lesbarkeit der weiteren Beschreibung wird diese anhand von Glykol als im Fehlerfall aus dem Aggregat 20 austretendem wassergefährdendem Medium fortgesetzt. Andere flüssige, wassergefährdende Stoffe, wie zum Beispiel Esteröle [?] oder dergleichen, sind im Folgenden bei jeder Erwähnung von Glykol jeweils mitzulesen und entsprechend gilt auch, dass mittels der als Glykolsensor 10 bezeichneten Einheit grundsätzlich auch andere wasserlösliche, wassergefährdende Stoffe sensierbar sind. Ebenso wird in der weiteren Beschreibung der Ultraschallsensor 10 als Glykolsensor 10 bezeichnet und auch hier ist bei jeder Erwähnung des Glykolsensors 10 die allgemeinere Bezeichnung als Ultraschallsensor 10 stets mitzulesen sowie mitzulesen, dass der Glykol-/Ultraschallsensor 10 aufgrund der Laufzeit-/Dichtemessung auch andere wasserlösliche und nicht wasserlösliche, insbesondere wassergefährdende Stoffe, zum Beispiel Öl, sensieren und bei deren Erkennung ein entsprechendes Signal generieren kann.

Im Falle einer Leckage wird das aus dem Aggregat 20 austretende Glykol von der Auffangwanne 14 aufgefangen und ein aufgrund des austretenden Glykols und/oder gegebenenfalls in sonstiger Weise zuströmender Flüssigkeit, zum Beispiel Regenwasser, resultierender Flüssigkeitsspiegel in der Auffangwanne 14 ist in der Darstellung in Figur 1 symbolisch in Form einer Wellenlinie gezeigt. Jedenfalls befindet sich bei einem Glykolaustritt Glykol oder ein Wasser-Glykol-Gemisch 22 in der Auffangwanne 14 und es besteht die Gefahr, dass das Wasser-Glykol-Gemisch 22 über einen Abfluss 24 oder einen Überlauf oder dergleichen aus der Auffangwanne 14 austritt und so ins Erdreich oder in umliegende Gewässer gelangt. Diese Gefahr besteht auch dann, wenn sich vor dem Abfluss 24 ein Abscheider 16 befindet, weil dieser nur nicht-wasserlösliche Stoffe, also zum Beispiel kein Glykol, abscheiden und zurückhalten kann.

Der Glykolsensor 10 sensiert (erkennt) im Betrieb die Anwesenheit von Glykol in der Auffangwanne 14 und steuert mittels eines Sensorsignals 26 gegebenenfalls das Ventil 18 oder einen sonstigen Aktor des Flüssigkeitsrückhaltesystems 12 an, welches bzw. welcher den Abfluss 24, einen Überlauf oder dergleichen der Auffangwanne 14 verschließt. Auf diese Weise wird der Austritt von Glykol oder eines jeweils sensierten wassergefährdenden Stoffes aus der Auffangwanne 14 sicher verhindert. Das Ventil 18 oder ein sonstiger Aktor kann sich im Innern der Auffangwanne 14 oder - wie dargestellt - außerhalb der Auffangwanne 14 befinden. Der Glykolsensor 10 ist so positioniert, dass er in Kontakt mit dem Wasser-Glykol-Gemisch 22 ist oder zumindest mit diesem in einen für eine Messung ausreichenden Kontakt kommt, sobald der Flüssigkeitsspiegel eine Höhe erreicht, bei der in absehbarer Zeit ein Austritt des Wasser-Glykol-Gemisches 22 über den Abfluss 24 möglich ist. Der Glykolsensor 10 kann also auf dem Boden der Auffangwanne 14 oder mit einem von der Höhe der Unterkante des Abflusses 24 abhängigen Abstand vom Boden der Auffangwanne 14 innerhalb des Flüssigkeitsrückhaltesystems 12 platziert sein und ist im letzteren Fall zum Beispiel am Rand der Auffangwanne 14 angebracht.

Ein in der Auffangwanne 14 platzierter (innerhalb der Grundfläche der Auffangwanne 14 platzierter) Glykolsensor 10 kann optional einen Feuchtigkeitssensor (nicht gezeigt) umfassen oder mit einem solchen Feuchtigkeitssensor kommunikativ kombiniert sein. Mittels des Feuchtigkeitssensors ist sensierbar, ob sich in der Auffangwanne 14 eine Flüssigkeit befindet. Auf Basis eines vom Feuchtigkeitssensor erhältlichen Feuchtigkeitssensor-Sensorsignals kann der Glykolsensor 10 optional deaktiviert werden, denn bei einer Auffangwanne 14, in der sich keine Flüssigkeit befindet, also bei einer "trockenen" Auffangwanne 14, kann sich auch kein Glykol oder dergleichen in der Auffangwanne 14 befinden, so dass der Betrieb des Glykolsensors 10 unnötig ist und dieser automatisch temporär deaktiviert werden kann. Sobald mittels des Feuchtigkeitssensors sensiert wird, dass sich Flüssigkeit in der Auffangwanne 14 befindet, wird der Glykolsensor 10 auf ein entsprechendes Feuchtigkeitssensor-Sensorsignal automatisch wieder aktiviert. Zusätzlich oder alternativ kann auf Basis eines vom Feuchtigkeitssensor erhältlichen Feuchtigkeitssensor-Sensorsignals auch ein eventuelles Heizelement in der Auffangwanne 14 und oder im Anschluss an die Auffangwanne 14 deaktiviert werden, denn bei einer "trockenen" Auffangwanne 14 ist auch keine Aufrechterhaltung einer Minimaltemperatur zur Gewährleistung einer Fließfähigkeit einer in der Auffangwanne 14 befindlichen Flüssigkeit notwendig.

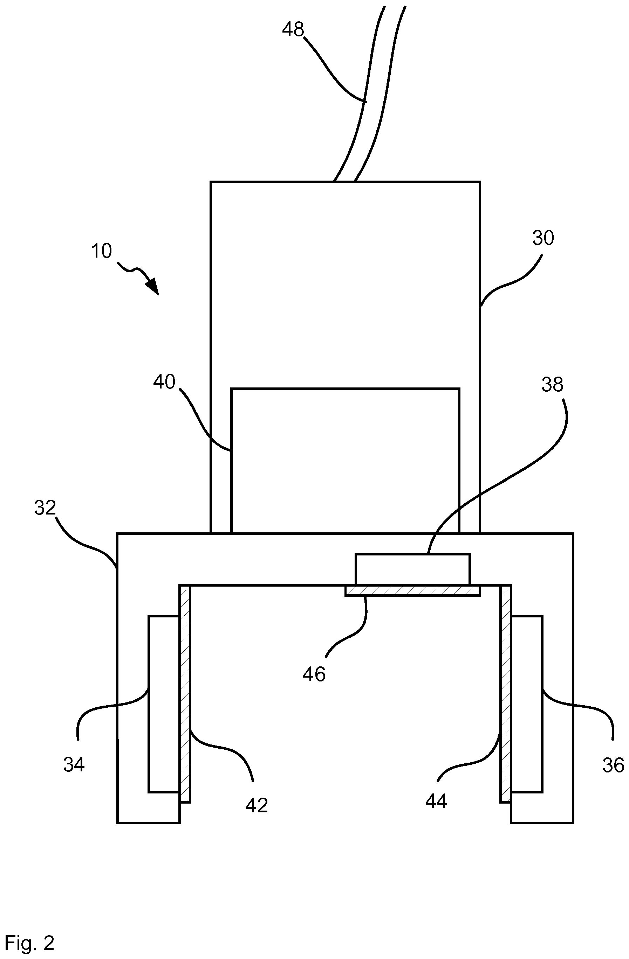

Die Darstellung in Figur 2 zeigt schematisch vereinfacht eine Ausführungsform eines Glykolsensors 10. Danach umfasst der Glykolsensor 10 in einer zum Beispiel gabelförmigen (U-förmigen), zu einem Gehäuse 30 gehörenden oder an das Gehäuse 30 anschließenden Halterung 32 einen im Folgenden kurz als Sender 34 bezeichneten Ultraschallsender, insbesondere einen als Sender 34 fungierenden piezokeramischen Ultraschallwandler, sowie einen entsprechend kurz als Empfänger 36 bezeichneten Ultraschallempfänger, insbesondere einen als Empfänger 36 fungierenden piezokeramischen Ultraschallwandler. Sender 34 und Empfänger 36 schließen eine freie Strecke von zum Beispiel 20 mm ein und beim Betrieb des Glykolsensors 10 befindet sich im Bereich dieser freien Strecke und damit zwischen Sender 34 und Empfänger 36 das jeweilige Medium, das auf eine Verunreinigung durch Glykol geprüft werden soll, also bei einer Situation gemäß Figur 1 das Wasser-Glykol-Gemisch 22. Für ein Wasser-Glykol-Gemisch 22 als zu prüfendes Medium wird die weitere Beschreibung - allerdings ausdrücklich ohne Verzicht auf eine weitergehende Allgemeingültigkeit - fortgesetzt. Andere Medien sind jeweils mitzulesen. Generell werden die Begriffe Flüssigkeit, Medium und Fluid zum Teil synonym genutzt: Aus der Auffangwanne 14 fließt eine Flüssigkeit, ein Fluid oder ein flüssiges Medium ab. Zusammen mit dieser bzw. diesem kann zum Beispiel ein wassergefährdender oder aus sonstigen Gründen rückhaltungsbedürftiger Stoff, insbesondere Glykol, aus der Auffangwanne 14 abfließen.

Der Abstand zwischen Sender 34 und Empfänger 36 ist aufgrund der Halterung 32 einerseits konstant und andererseits bekannt. Beim Betrieb des Glykolsensors 10 wird die Zeit gemessen (Laufzeit), die ein vom Sender 34 ausgesandtes Signal bis zur Detektion durch den Empfänger 36 benötigt. Mittels eines Temperatursensors 38 wird die jeweilige Temperatur des Wasser-Glykol-Gemisches 22 erfasst. Bei der gezeigten Ausführungsform ist der Temperatursensor 38 als an der Halterung 32 angebracht dargestellt. Der Temperatursensor 38 kann auch räumlich vom Glykolsensor 10 beabstandet angeordnet sein (zum Beispiel indem der Temperatursensor 38 unabhängig vom Glykolsensor 10 an der Auffangwanne 14 angebracht ist). Wesentlich ist, dass der Temperatursensor 38 in Kontakt mit demselben Medium ist, dem der Glykolsensor 10 ausgesetzt ist, und dass eine Steuerungseinheit 40 des Glykolsensors 10 kommunikativ sowohl mit dem Sender 34 und dem Empfänger 36 wie auch mit dem Temperatursensor 38 verbunden ist.

Optional befindet sich auf der zur freien Strecke zwischen Sender 34 und Empfänger 36 weisenden Oberfläche des Senders 34 und/oder Empfängers 36 eine Schutzschicht 42, 44, mittels derer der Sender 34 bzw. der Empfänger 36 vor Beschädigungen durch chemisch aggressive Stoffe im jeweiligen Medium und/oder vor mechanischen Beschädigungen geschützt wird. Optional ist auch eine entsprechende Oberfläche des Temperatursensors 38 mittels einer eigenen Schutzschicht 46 in gleicher Weise geschützt. Zur Versorgung mit elektrischer Energie und zur Weiterleitung eines mittels des Glykolsensors 10 generierbaren Sensorsignals 26 ist zum entsprechenden Anschluss des Glykolsensors 10 in grundsätzlich an sich bekannter Art und Weise zumindest ein Anschlusskabel 48 vorgesehen.

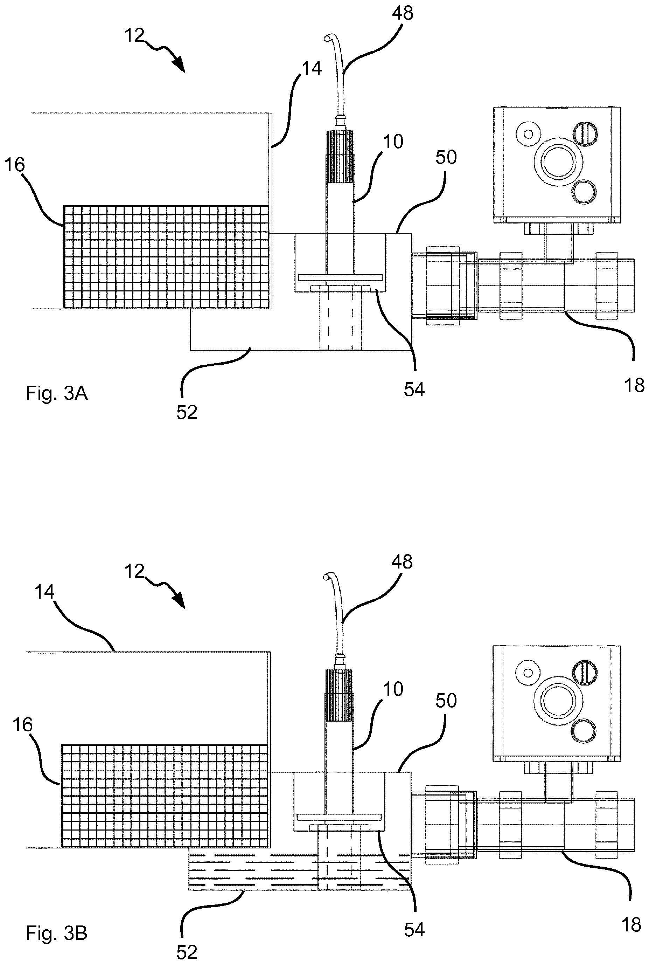

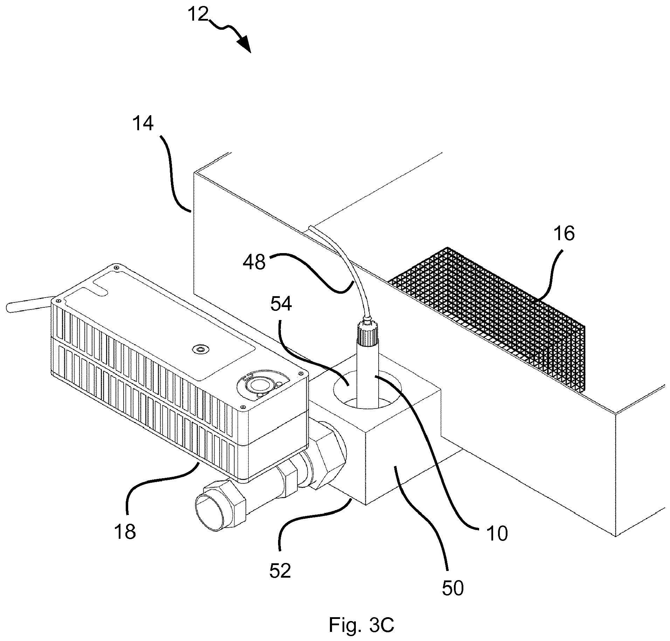

Die Darstellung in Figur 3 (Fig. 3A, 3B, 3C) zeigt, dass der Glykolsensor 10 im Gegensatz zu der in Figur 1 exemplarisch gezeigten Situation genauso auch außerhalb der Auffangwanne 14, also zum Beispiel im Anschluss an einen Abfluss 24 aus der Auffangwanne 14 angeordnet sein kann. Die Darstellung in Figur 3A und 3B zeigt insoweit in einer Seitenansicht von links nach rechts einen Teil einer Auffangwanne 14 mit einem die Auffangwanne 14 seitlich begrenzenden Rand, wobei sich randseitig in der Auffangwanne 14 oder optional beabstandet von dem Rand ein Abscheider 16, zum Beispiel ein Abscheider in Form einer Flüssigkeitsrückhaltevorrichtung 16 gemäß der EP 2 335 794 B oder der EP 2 716 827 B, befindet. Bei dem Abscheider 16 kann es sich im einfachsten Fall auch um ein Gitter oder dergleichen handeln, welches zum Beispiel einen Abfluss von Laub oder dergleichen aus der Auffangwanne 14 verhindert.

An einen als Abfluss 24 aus der Auffangwanne 14 fungierenden Auslass aus dem Abscheider 16 oder allgemein stromabwärts des Auslasses ist eine Sensoraufnahmevorrichtung 50 angeschlossen, in welcher sich stromabwärts der Auffangwanne 14 ein Glykolsensor 10 befindet. Der Glykolsensor 10 ist in der Sensoraufnahmevorrichtung 50 vertikal zu einer Längsachse der Sensoraufnahmevorrichtung 50 und damit vertikal zu einer Flussrichtung eines jeweiligen Mediums durch die Sensoraufnahmevorrichtung 50 eingebaut. Die Sensoraufnahmevorrichtung 50 weist einen unterhalb des Bodens der Auffangwanne 14 liegen Bodenabschnitt 52 auf. Im Anschluss an den tiefer liegenden Bodenabschnitt 52 oder im Anschluss an die Sensoraufnahmevorrichtung 50 herrscht ein Niveau, das einen Abfluss eines in der Auffangwanne 14 befindlichen Fluids aus der Auffangwanne 14 erlaubt. Der lokal tiefer liegende Bodenabschnitt 52 führt dazu, dass sich in diesem Bereich - ähnlich wie bei einem Siphon -aus der Auffangwanne 14 abfließendes Fluid sammelt.

Die Darstellung in Figur 3B zeigt eine Sensoraufnahmevorrichtung 50 gemäß Figur 3A, die bis zum Bodenniveau der Auffangwanne 14 und dem Niveau der weiterführenden Rohrleitung mit einem Fluid gefüllt ist, welches zuvor aus der Auffangwanne 14 abgeflossen ist. Der lokal tiefer liegende Bodenabschnitt 52 definiert ein Becken, in dem Sender 34 und Empfänger 36 des Glykolsensors 10 ganz oder zumindest im Wesentlich in das dort zurückgehaltene Fluid eingetaucht sind. Diese gewissermaßen "tiefergelegte" Position des Glykolsensors 10 führt dazu, dass auch bei einer nur geringen Menge von aus der Auffangwanne 14 abfließendem Fluid Sender 34 und Empfänger 36 des Glykolsensors 10 ganz oder zumindest im Wesentlichen in das Fluid eingetaucht sind. Dies führt zu verlässlicheren Messergebnissen. Bei einem nicht tiefer liegenden Glykolsensor 10 und bei einer nur geringen Menge von aus der Auffangwanne abfließendem Fluid werden Sender und Empfänger 34 des Glykolsensors 10 möglicherweise gar nicht mit dem Fluid in Kontakt oder es ist nur ein vergleichsweise kleiner Abschnitt des Senders 34 und des Empfängers 36 in das Fluid eingetaucht. Zumindest die verbleibenden Abschnitte von Sender und Empfänger 34, 36 sind nicht von dem Fluid benetzt. Mittels des Empfängers 36 werden folglich bei zumindest teilweise eingetauchtem Sender und Empfänger 34, 36 einerseits Ultraschallsignale detektiert, deren Laufzeit durch das Fluid bestimmt ist und andererseits werden Ultraschallsignale detektiert, deren Laufzeit nicht durch das Fluid reduziert wurde, so dass die Laufzeit vielmehr durch die Umgebungsluft bestimmt ist. Eine verlässliche Dichtemessung des Fluids ist damit zumindest erschwert und ohne eine verlässliche Dichtemessung ist auch eine Sensierung von in dem Fluid enthaltenem Glykol zumindest erschwert. Bei der tiefer liegenden Position des Glykolsensors 10 sind Sender und Empfänger 34, 36 ganz oder zumindest im Wesentlichen in das aus der Auffangwanne 14 stammende Fluid eingetaucht, sodass ein Sensorsignal 26 vom Glykolsensor 10 verlässlich die Dichte des Fluids im Bereich des Glykolsensors 10 repräsentiert und folglich die Sensierung von in dem Fluid enthaltenem Glykol erlaubt.

Die Sensoraufnahmevorrichtung 50 umfasst bei der in Figur 3 gezeigten Ausführungsform zur Aufnahme des Glykolsensors 10 eine von der Hüllfläche der Sensoraufnahmevorrichtung 50 zurückgesetzte Einstülpung 54. Bei der Einstülpung 54 kann es sich zum Beispiel um eine zylindrische Einstülpung 54 oder eine Einstülpung 54 mit einer polygonalen Grundfläche handeln. Der Glykolsensor 10 ist am Boden der Einstülpung 54 fixiert, zum Beispiel indem der Glykolsensor 10 mit einem Gewinde an dessen Außenoberfläche in ein Gewinde in der Bodenfläche der Einstülpung 54 eingeschraubt ist. Die Einstülpung 54 schützt den Glykolsensor 10, insbesondere den Abschnitt des Glykolsensors 10 mit der Steuerungseinheit 40, (siehe Figur 2) vor mechanischen Beschädigungen.

Die Darstellung in Figur 3C zeigt das Flüssigkeitsrückhaltesystem 12, die Sensoraufnahmevorrichtung 50 und den Glykolsensor 10 in einer isometrischen Ansicht. In dieser Darstellung wird der mechanische Schutz des Glykolsensors 10 durch die Sensoraufnahmevorrichtung 50 besonders deutlich.

Ein Sensorsignal 26 vom Glykolsensor 10 wird über dessen Anschlusskabel 48 vom Glykolsensor 10 fortgeleitet und beispielsweise direkt einem Ventil 18 oder einem sonstigen Aktor zum Verhindern eines weiteren Abflusses von Fluid aus der Auffangwanne 14 zugeleitet. Genauso kann das Sensorsignal 26 - und gegebenenfalls ein Sensorsignal 26 von zumindest einem weiteren Glykolsensor 10 - einer zentralen Verarbeitungseinheit zugeleitet werden, welche als Ergebnis einer Verarbeitung des Sensorsignals 26 ein Ansteuersignal zur Ansteuerung eines Ventils 18, das in den Figuren mit seinem Ventilantrieb gezeigt ist, oder eines sonstigen Aktors oder einer Gruppe solcher Ventile oder Aktoren generiert.

Bevorzugt, aber grundsätzlich optional, erfolgt die Verarbeitung der mittels der Sensorik - Sender und Empfänger 34, 36 oder Sender, Empfänger und Temperatursensor 34, 36, 38 - eines Glykolsensors 10 aufgenommenen Daten vollständig durch den Glykolsensor 10 und dessen Steuerungseinheit 40 selbst. Der Glykolsensor 10 liefert damit als Sensorsignal 26 zumindest ein Binärsignal. Ein erster Status des Binärsignals kodiert zum Beispiel, dass kein Glykol sensiert wurde oder nur eine Glykolkonzentration unterhalb eines vorgegebenen oder vorgebbaren Schwellwerts sensiert wurde. Ein komplementärer zweiter Status des Binärsignals kodiert entsprechend, dass Glykol sensiert wurde oder eine Glykolkonzentration oberhalb des Schwellwerts sensiert wurde. Ein solches mittels der Steuerungseinheit 40 des Glykolsensor 10 generierbares und im Betrieb generiertes Binärsignal erfordert nachfolgend, zum Beispiel bei einer zentralen Verarbeitungseinheit, keine weitere Verarbeitung und auf den dortigen Empfang eines Sensorsignals 26, welches kodiert, dass Glykol sensiert wurde, kann unmittelbar ein Steuersignal zur Ansteuerung eines Ventils18 oder eines sonstigen Aktors oder einer Gruppe solcher Ventile oder Aktoren generiert werden. Genauso kann ein solches digitales Sensorsignal 26 einem entsprechend eingerichteten Ventil 18 oder sonstigem Aktor direkt zugeführt werden und fungiert dort wie ein ansonsten von einer zentralen Verarbeitungseinheit generiertes Steuersignal. Das vom Glykolsensor 10 und dessen Steuerungseinheit 40 generierte Digitalsignal wird über dessen Anschlusskabel 48 übermittelt, zum Beispiel über eine vom Anschlusskabel 48 umfasste Signalleitung oder vom Anschlusskabel 48 umfasste Busleitungen. Alternativ kann der Glykolsensor 10 auch parallel zu dem Anschlusskabel 48 eine weitere vom und zum Glykolsensor 10 führende Leitung aufweisen, welche eine solche Signalleitung und/oder Busleitungen umfasst. Optional kann der Glykolsensor 10 und dessen Steuerungseinheit 40 zusätzlich zu dem Binärsignal auch ein digitales oder analoges Signal generieren und über eine Signalleitung oder Busleitungen aussenden, welches eine jeweils erfasste Glykolkonzentration kodiert und damit über das Binärsignal hinaus zusätzliche Informationen zum Beispiel für eine zentrale Verarbeitungseinheit verfügbar macht.

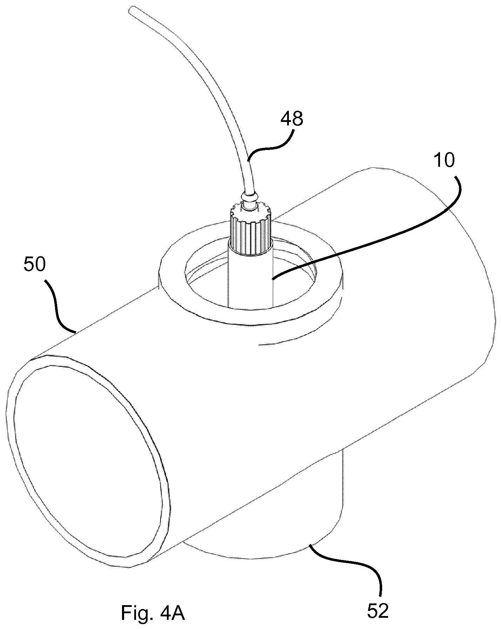

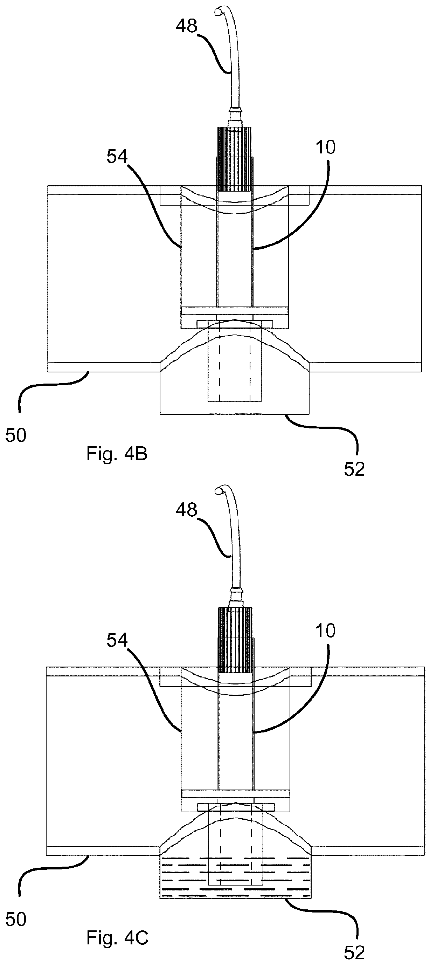

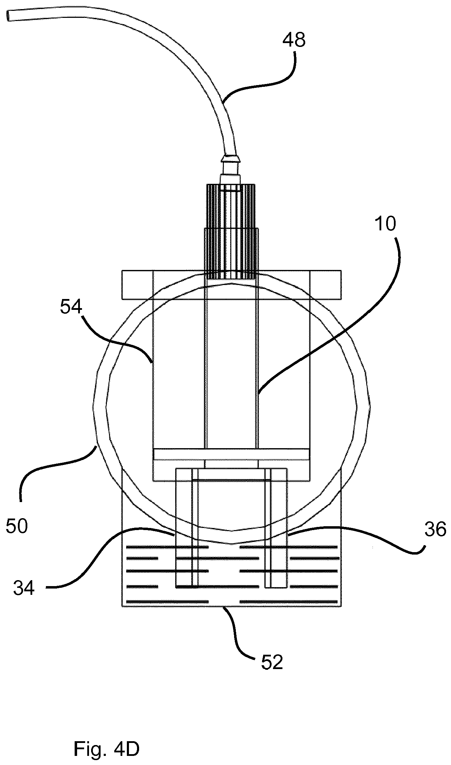

Die Darstellungen in Figur 4 (Figur 4A, 4B, 4C, 4D) zeigen eine alternative Ausführungsform einer Sensoraufnahmevorrichtung 50. Dabei fungiert als Sensoraufnahmevorrichtung 50 ein Rohrabschnitt. Zum Einsetzen einer solchen Sensoraufnahmevorrichtung 50 in eine Rohrleitung kann die Sensoraufnahmevorrichtung 50 an beiden offenen Enden einen Flansch oder dergleichen (nicht gezeigt) zum Verbinden mit einer ankommenden und einer abgehenden Rohrleitung aufweisen. Der tiefer liegende Bodenabschnitt 52 liegt in Relation zu einer Sohle des beidseitig angrenzenden Rohrabschnitts tiefer. Bei der gezeigten Ausführungsform ist der tieferliegende Bodenabschnitt 52 das untere Ende einer zylindrischen Ausstülpung der Sensoraufnahmevorrichtung 50. Grundsätzlich kommen auch andere Geometrien zum Erhalt des tiefer liegenden Bodenabschnitts 52 in Betracht, zum Beispiel eine Ausstülpung mit einer polygonalen Grundfläche. Die Darstellung in Figur 4A zeigt diese Ausführungsform der Sensoraufnahmevorrichtung 50 in einer isometrischen Ansicht. Die Figuren 4B und 4C zeigen diese Ausführungsform in einer entlang der Längsachse der Sensoraufnahmevorrichtung 50 geschnittenen Seitenansicht. In der Darstellung in Figur 4C ist in dem als Reservoir fungierenden Bodenabschnitt 52 ein Teil eines aus der Auffangwanne 14 ausgeströmten Fluids zurückgehalten und benetzt Sender und Empfänger 34, 36 des Glykolsensors 10. Figur 4D zeigt diese Ausführungsform in einer quer zur Längsachse der Sensoraufnahmevorrichtung 50 geschnittenen Seitenansicht. Speziell in der Darstellung in Figur 4C sind Sender und Empfänger 34, 36 des Glykolsensors 10 erkennbar und auch bei der in Figur 4C gezeigten Situation ist erkennbar, dass im Bodenabschnitt 52 ein Teil eines aus der Auffangwanne 14 ausgeströmten Fluids zurückgehalten ist und dass Sender und Empfänger 34, 36 in das zurückgehaltene Fluid eingetaucht sind.

Die Darstellung in Figur 4 zeigt, dass der Glykolsensor 10 von der Einstülpung 54 nahezu vollständig aufgenommen ist. Eine solche oder eine vollständige Aufnahme gewährleistet einen noch besseren Schutz gegen mechanische Beschädigungen oder dergleichen. Eine solche Aufnahme kann auch bei der Ausführungsform gemäß Figur 3 vorgesehen sein und hängt letztlich von der Dimension des Glykolsensors 10 und der Dimension der Sensoraufnahmevorrichtung 50 ab.

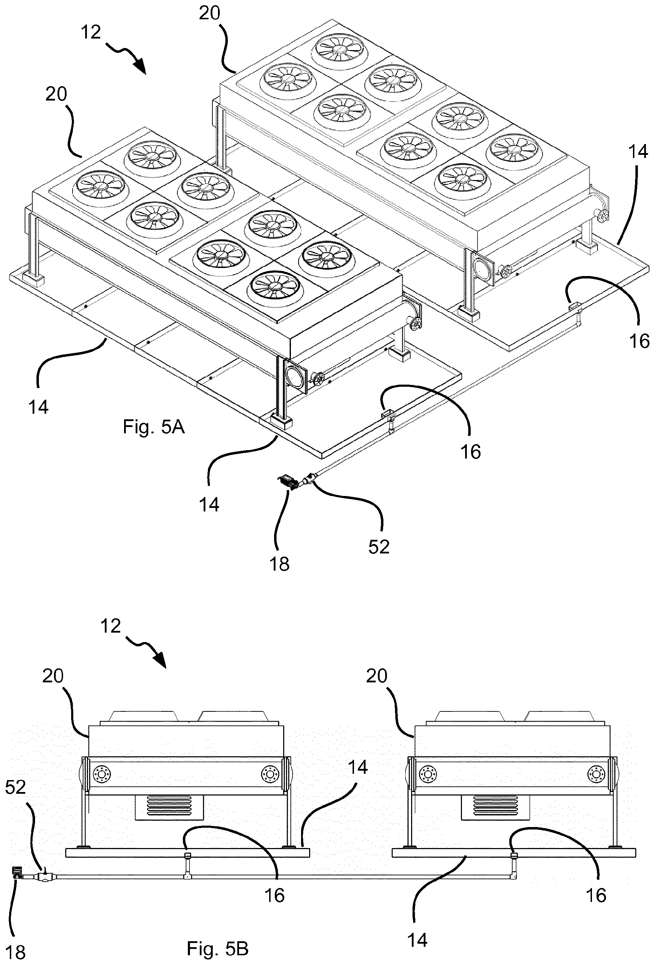

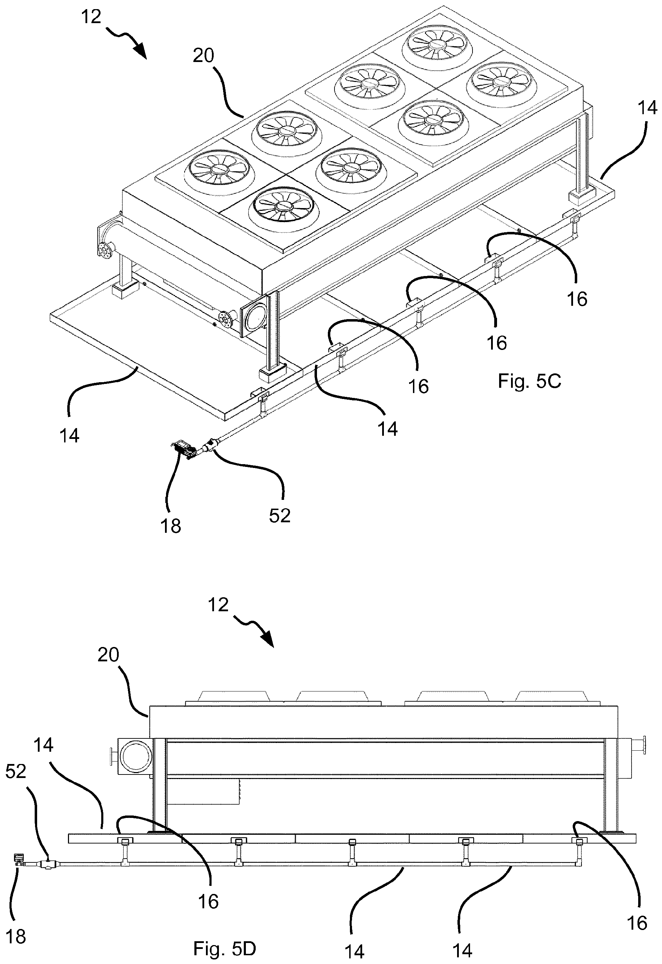

Die Darstellung in Figur 5 (Figur 5A, 5B, 5C, 5D) zeigt, dass auch die Sensoraufnahmevorrichtung 50 gemäß Figur 4 mit einem darin angebrachten Glykolsensor 10 stromabwärts einer Auffangwanne 14 (oder mehrerer Auffangwannen 14) angebracht ist und dafür an eine von einer Auffangwanne 14 kommende Rohrleitung und stromaufwärts eines Ventils 18 oder dergleichen in derselben Rohrleitung angeschlossen wird oder ist. Auch bei der Ausführungsform gemäß Figur 4 wird im Bereich des tiefer liegenden Bodenabschnitts 52 aus der Auffangwanne 14 abfließendes Fluid zurückgehalten, sodass Sender und Empfänger 34, 36 des Glykolsensors 10 auch bei einer geringen Menge von aus der Auffangwanne 14 abfließendem Fluid ganz oder zumindest im Wesentlichen in das Fluid eingetaucht sind.

Figur 5A und 5B zeigen ein Flüssigkeitsrückhaltesystem 12 gemäß Figur 1, jedoch anders als in Figur 1 nicht so stark schematisch vereinfacht und mit realistischen Größenverhältnissen. Unter den Aggregaten 20 der leckagegefährdeten Anlage befinden sich mehrere miteinander verbundene und fluidisch gekoppelte Auffangwannen 14. An eine äußere Auffangwanne 14 ist eine Rohrleitung angeschlossen, in welcher sich die Sensoraufnahmevorrichtung 50 und ein darin platzierter Glykolsensor 10 befinden. Die Sensoraufnahmevorrichtung 50 ist stromaufwärts an ein von der Auffangwanne 14 kommendes Rohrleitungsstück und stromabwärts an ein weiteres Rohrleitungsstück angeschlossen. Die Darstellung in Figur 5A zeigt eine isometrische Ansicht des Flüssigkeitsrückhaltesystems 12. Die Darstellung in Figur 5B zeigt eine Seitenansicht des Flüssigkeitsrückhaltesystems 12. Stromabwärts der Sensoraufnahmevorrichtung 50 befindet sich ein Ventil 18 oder ein sonstiger zum Verschließen der von der Auffangwanne 14 kommenden Rohrleitung geeigneter Aktor. Das Ventil 18 oder der Aktor werden auf Basis eines von dem Glykolsensor 10 der Sensoraufnahmevorrichtung 50 stammenden Sensorsignals 26 angesteuert.

Figur 5C und 5D zeigen ein Flüssigkeitsrückhaltesystem 12 ähnlich wie in Figur 5A und 5B. Im Unterschied "entwässern" hier jedoch die Auffangwannen 14 einzeln in eine Rohrleitung, in der sich stromabwärts der Auffangwannen 14 eine Sensoraufnahmevorrichtung 50 mit einem darin platzierten Glykolsensor 10 befindet. Stromabwärts der Sensoraufnahmevorrichtung 50 befindet sich ein Ventil oder ein sonstiger zum Verschließen der von der Auffangwanne 14 kommenden Rohrleitung geeigneter Aktor. Auch hier sind das Ventil 18 oder der Aktor auf Basis eines von den Glykolsensor 10 der Sensoraufnahmevorrichtung 50 stammenden Sensorsignals 26 ansteuerbar.

Die weitere Beschreibung wendet sich jetzt wieder dem Glykolsensor 10 und dessen Funktion zu.

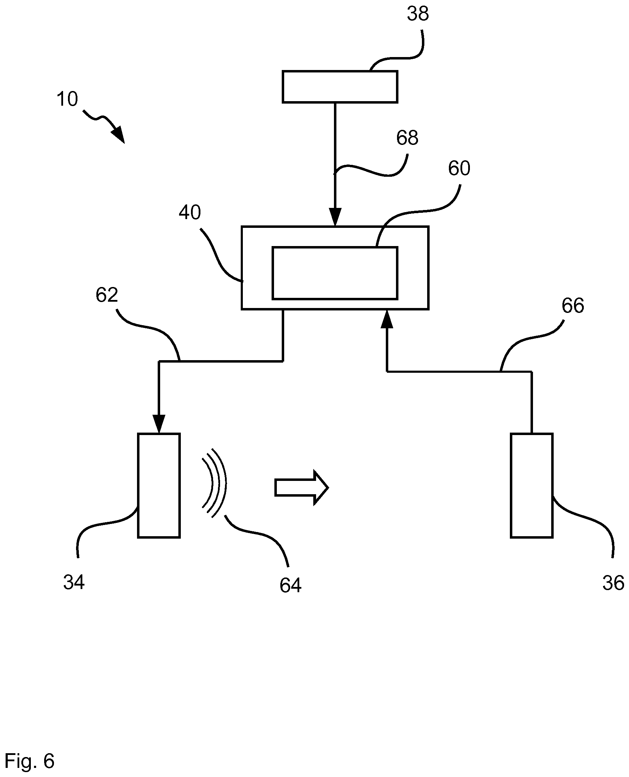

Die Darstellung in Figur 6 zeigt den Glykolsensor 10 gemäß Figur 1 in einer auf dessen wesentliche Funktionseinheiten reduzierten Form. Die Steuerungseinheit 40 umfasst in grundsätzlich an sich bekannter Art und Weise einen Speicher (nicht gezeigt), in den ein Steuerungsprogramm 60 ladbar ist, welches beim Betrieb des Glykolsensors 10 mittels eines von der Steuerungseinheit 40 umfassten Mikroprozessors (nicht gezeigt) oder dergleichen ausgeführt wird. Das Steuerungsprogramm 60 umfasst Programmcodeanweisungen, welche die Funktionalität des Glykolsensors 10 bestimmen, und wenn im Folgenden einzelne mittels des Glykolsensors 10 ausgeführte Verfahrensschritte erläutert werden, handelt es sich um Verfahrensschritte, welche automatisch gemäß dem Steuerungsprogramm 60 und unter Kontrolle der Steuerungseinheit 40 ablaufen.

Zum Messen des Glykolgehalts des Wasser-Glykol-Gemisches 22 in der Auffangwanne 14 oder im Bodenabschnitt 52 der Sensoraufnahmevorrichtung 50 aktiviert (Startsignal 62) die Steuerungseinheit 40 zu vorgegebenen oder vorgebbaren, insbesondere äquidistanten Zeitpunkten den Sender 34, der darauf ein Signal, im Falle eines Ultraschallsenders ein Ultraschallsignal 64, in Richtung auf dem Empfänger 36 abstrahlt (wie durch den Blockpfeil illustriert). Beim Empfang des Ultraschallsignals 64 durch den Empfänger 36 erzeugt dieser ein Empfangssignal 66 für die Steuerungseinheit 40. Mit dem Aussenden des Startsignals 62 wird ein Zeitgeber (nicht gezeigt), zum Beispiel ein als Teil des Steuerungsprogramms 60 in Software implementierter Zähler oder ein Hardware-Zähler, gestartet. Mit dem Eingang des Empfangssignals 66 wird der Zeitgeber gestoppt. Der resultierende Zustand des Zeitgebers ist ein Maß für die vom Ultraschallsignal 64 für die Zurücklegung der Strecke zwischen Sender 34 und Empfänger 36 benötigte Zeit (Laufzeit) und bei der Ermittlung der Laufzeit handelt es sich entsprechend um eine Laufzeitmessung. In Abhängigkeit von der jeweiligen Implementierung des Zeitgebers, bei einem Zähler zum Beispiel in Abhängigkeit von einer Frequenz, mittels derer dieser getaktet wird, kann in grundsätzlich an sich bekannter Art und Weise auf Basis des beim Eingang des Empfangssignals 66 gegebenen Zustands des Zeitgebers ein tatsächlicher Zeitwert ermittelt werden. Mit diesem Zeitwert und der bekannten Länge der Strecke zwischen Sender 34 und Empfänger 36 kann schließlich die Geschwindigkeit (Schallgeschwindigkeit) des Ultraschallsignals 64 in dem jeweiligen Medium, vorliegend also in dem Wasser-Glykol-Gemisch 22 in der Auffangwanne 14 oder im Bodenabschnitt 52 der Sensoraufnahmevorrichtung 50, berechnet werden. Die resultierende Geschwindigkeit (aber auch schon die zugrunde liegende Laufzeit sowie der wiederum dieser zugrunde liegende Zustand des Zeitgebers) gibt einen Anhalt dafür, ob eine Verunreinigung mit Glykol vorliegt oder nicht.

Im Interesse einer besseren Lesbarkeit der nachfolgenden Beschreibung wird diese zunächst anhand einer jeweils mittels des Glykolsensors 10 ermittelten Geschwindigkeit (Schallgeschwindigkeit) fortgesetzt. Die Verwendbarkeit der zugrunde liegenden Laufzeit sowie die Verwendbarkeit des dieser wiederum zugrunde liegenden Zustands des Zeitgebers, insbesondere eines Zählerstands, ist jeweils mitzulesen und als von der hier vorgelegten Beschreibung mit umfasst anzusehen.

Bekanntlich ist die Geschwindigkeit einer Schallwelle in einem Medium (Schallgeschwindigkeit) unter anderem abhängig von der jeweiligen Dichte des Mediums. Die Dichte von Glykol ist mit ca 1,1 g/cm⁻³ geringfügig höher als die Dichte von Wasser (ca. 1,00 g/cm⁻³). Die Dichte eines Wasser-Glykol-Gemisches ist also höher als die Dichte von Wasser. Die Dichte eines Wasser-Glykol-Gemisches steigt also mit der enthaltenen Glykolmenge. Wenn aus dem jeweiligen Aggregat 20 (Fig. 1, Fig. 5) einer leckagegefährdeten Anlage Glykol austritt und sich mit Wasser in einer Auffangwanne 14 unterhalb der Anlage mischt, ist die Dichte des resultierenden Wasser-Glykol-Gemisches 22 höher als die Dichte von Wasser, wobei der Abstand zur Dichte von Wasser abhängig von der ausgetretenen Glykolmenge ist. Durch Vergleich der in einem solchen Wasser-Glykol-Gemisch 22 resultierenden Schallgeschwindigkeit mit der (bekannten) Schallgeschwindigkeit in Wasser (Leitungswasser, Regenwasser, Brackwasser und dergleichen) lässt sich automatisch ermitteln, ob ein Glykolaustritt vorliegt.

Bekanntlich ist die Dichte einer Flüssigkeit oder eines Gemisches mehrerer Flüssigkeiten aber auch von deren bzw. dessen Temperatur abhängig, so dass die Genauigkeit der laufzeitbasierten Erkennung eines Glykolaustritts von der jeweiligen Temperatur des Mediums, die üblicherweise der Umgebungstemperatur entspricht, abhängt. Eine jeweilige Dichte und eine damit korrelierte Schallgeschwindigkeit sind also nicht allein aussagekräftig, wenn es darum geht, einen Glykolaustritt sicher zu erkennen. Darum ist bei dem hier vorgeschlagenen Glykolsensor 10 eine Temperaturkompensation vorgesehen. Dafür erhält die Steuerungseinheit 40 vom Temperatursensor 38 ein die jeweilige Temperatur des Mediums im Bereich des Glykolsensors 10 kodierendes Temperatursignal 68.

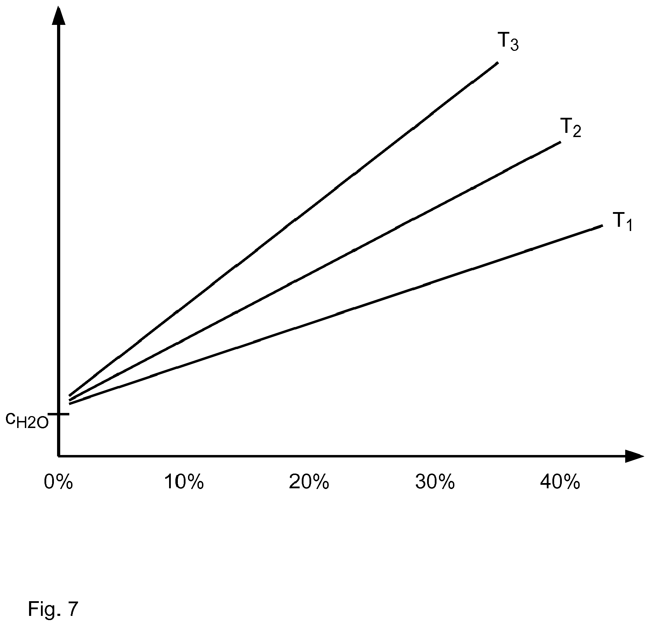

In der Darstellung in Figur 7 sind in dem dort gezeigten Koordinatensystem auf der Abszisse eine Glykolkonzentration in Wasser in Prozent und auf der Ordinate die Schallgeschwindigkeit aufgetragen. Die Schallgeschwindigkeit in (nicht verunreinigtem) Wasser ist links unten unmittelbar auf der Ordinate (und damit bei 0% Glykolkonzentration) mit C_{H2O} eingezeichnet. Anhand der in dem Koordinatensystem eingezeichneten Kurvenschar ist erkennbar, dass mit zunehmender Glykolkonzentration die in dem resultierenden Wasser-Glykol-Gemisch 22 gemessene Schallgeschwindigkeit ansteigt und jedenfalls oberhalb von C_{H2O} liegt. Die gezeigten Kurven der Kurvenschar sind für unterschiedliche Temperaturen aufgenommen, die in der Darstellung symbolisch mit T₁, T₂ und T₃ (T₁ < T₂ < T₃) angegeben sind. Es ist darauf hinzuweisen, dass der Verlauf der eingezeichneten Kurven ein idealisierter Verlauf ist und hier nur zur Veranschaulichung dient. Auch die Anzahl der gezeigten Kurven ist lediglich beispielhaft zu verstehen und in der Praxis können mehr oder weniger als drei Kurven berücksichtigt werden. Zudem ist stets auch eine Interpolation zwischen benachbarten Kurven möglich.

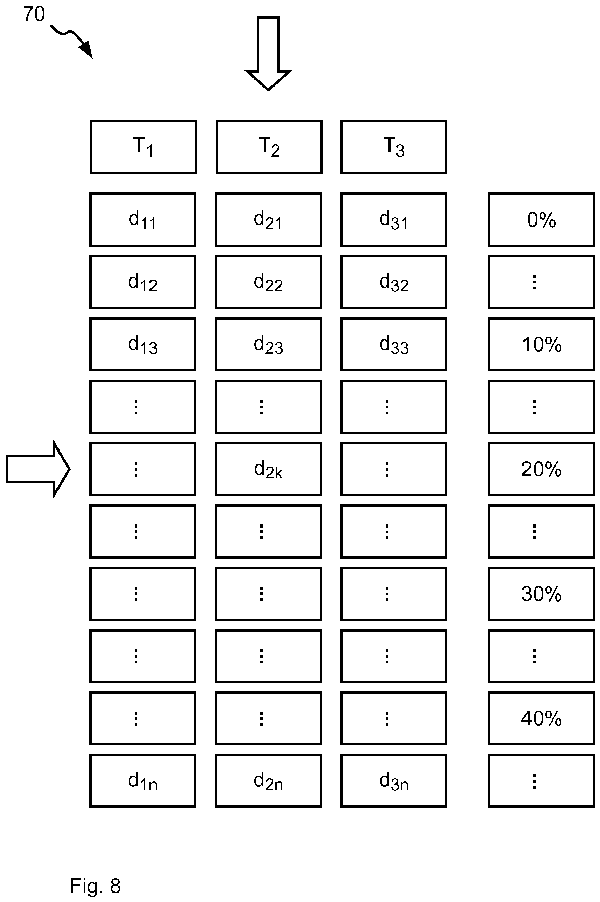

Die solchen Kurven zugrunde liegenden Daten sind im Speicher der Steuerungseinheit 40 des Glykolsensors 10 gespeichert, zum Beispiel in Tabellenform. Figur 8 zeigt eine graphische Darstellung einer solchen als Umsetzungstabelle 70 fungierenden Tabelle und der darin enthaltenen Daten. Die weitere Beschreibung erfolgt anhand der gezeigten Situation mit in einzelnen Spalten für die jeweiligen Temperaturen T₁, T₂ und T₃ zusammengefassten Daten. Selbstverständlich ist auch eine Form der im Folgenden kurz als Tabelle 70 bezeichneten Umsetzungstabelle 70 möglich, bei der die zu den Temperaturen T₁, T₂ und T₃ gehörigen Daten jeweils in einzelnen Zeilen zusammengefasst sind. Dies ist stets mitzulesen.

Gemäß Figur 8 umfasst die Tabelle 70 eine Mehrzahl von Spalten für unterschiedliche Temperaturen T₁, T₂ und T₃ sowie eine Mehrzahl von Zeilen für unterschiedliche Glykolkonzentrationen. Die mit "T₁", "T₂" und "T₃" bezeichneten Elemente sind als "Spaltenüberschriften" anzusehen und nicht oder zumindest nicht notwendig Teil der Tabellendaten. Ähnlich sind die mit "0%", "10%" usw. bezeichneten Elemente als "Zeilenüberschriften" anzusehen und ebenfalls nicht oder zumindest nicht notwendig Teil der Tabellendaten. Die eigentlichen Tabellendaten "d₁₁" bis "d₃ₙ" umfassen jeweils Schallgeschwindigkeits-Messwerte und zwar Schallgeschwindigkeits-Messwerte bei einer bestimmten Temperatur und einer bestimmten Glykolkonzentration. Zum Beispiel ist der in der Darstellung in Figur 8 mit "d₁₃" bezeichnete Schallgeschwindigkeits-Messwert bei der Temperatur "T₁" und einer Glykolkonzentration von 10% aufgenommen worden. Entsprechend sind die in der Darstellung in Figur 8 mit "d₂₃" und "d₃₃" bezeichneten Schallgeschwindigkeits-Messwerte bei der Temperatur "T₂" bzw. der Temperatur "T₃" und jeweils einer Glykolkonzentration von 10% aufgenommen worden. Alle bei der Temperatur T₁ aufgenommenen Schallgeschwindigkeits-Messwerte ("d₁₁" bis "d₁ₙ") sind in der Spalte für die Temperatur "T₁" und alle bei der Temperatur T₂ aufgenommenen Schallgeschwindigkeits-Messwerte ("d₂₁" bis "d₂ₙ") sind in der Spalte für die Temperatur "T₂" zusammengefasst usw. In der jeweiligen Spalte sind die einzelnen Schallgeschwindigkeits-Messwerte jeweils in der Zeile mit der betreffenden Glykolkonzentration eingetragen, bei welcher der Schallgeschwindigkeit-Messwert ursprünglich aufgenommen wurde. Der Inhalt der Tabelle 70 wird also dem Speicher der Steuerungseinheit 40 auf Basis empirisch aufgenommener Schallgeschwindigkeits-Messwerte eingeprägt, bevor der Glykolsensor 10 in Betrieb geht. Anstelle von Schallgeschwindigkeits-Messwerten kann die Tabelle 70 auch die einem berechneten Schallgeschwindigkeits-Messwert jeweils zugrunde liegenden Laufzeiten oder sogar einen Zählerstand enthalten. Welche Daten tatsächlich in der Tabelle 70 gespeichert sind, hängt davon ab, welcher Vergleichswert beim späteren Betrieb des Glykolsensors 10 zum Sensieren eines eventuellen Glykolaustritts verwendet wird.

Beim Betrieb des Glykolsensors 10 wird das jeweilige Medium, also zum Beispiel das in einer Auffangwanne 14 unter einem leckagegefährdeten Aggregat 20 befindliche Wasser oder ein eventuelles Wasser-Glykol-Gemisch 22, zyklisch durch Aussenden von Ultraschallsignalen 64 untersucht und parallel dazu jeweils anhand eines Temperatursignals 68 ein Temperaturmesswert ermittelt. Beim Empfang eines Ultraschallsignals 64, welches durch das jeweilige Medium hindurchgelaufen ist, ergibt sich ein Zustand des Zeitgebers, insbesondere ein Zählerstand, eine daraus berechnete Laufzeit oder eine wiederum daraus berechnete Schallgeschwindigkeit. Jedes dieser Ergebnisse kommt für die weitere Verwendung in Betracht und - wie oben bereits grundsätzlich erwähnt - hängt vom Steuerungsprogramm 60 der Steuerungseinheit 40 ab, welche Daten in der Tabelle 70 gespeichert sind (Zählerstände, Laufzeiten, Schallgeschwindigkeiten). Im Folgenden werden ein aufgrund eines ausgesandten und empfangenen Ultraschallsignals 64 resultierender Zustand des Zeitgebers oder eine berechnete Laufzeit oder eine berechnete Schallgeschwindigkeit einzeln und zusammen als Messergebnis bezeichnet.

Mit dem jeweils ermittelten Temperaturmesswert und dem Messergebnis erfolgt der Zugriff auf die Tabelle 70. Dies ist in der Darstellung in Figur 8 symbolisch mittels zweier Blockpfeile veranschaulicht. Ein erster, abwärts gewandter Blockpfeil ist über der Spalte für die Temperatur "T₂" gezeigt und allgemein erfolgt die Auswahl einer der Spalten der Tabelle 70 anhand des jeweiligen Temperaturmesswerts. Ein zweiter, nach rechts gewandter Blockpfeil ist neben einer der Zeilen der Tabelle 70 gezeigt und allgemein ergibt sich die jeweilige Zeile der Tabelle 70 anhand des Messergebnisses, zum Beispiel indem der in der bereits anhand des Temperaturmesswerts ausgewählten Spalte befindliche Zahlenwert innerhalb vorgegebener oder vorgebbarer Grenzen mit dem jeweiligen Messergebnis übereinstimmt. Sobald auf diese Weise die zu dem Temperaturmesswert und dem Messergebnis gehörige Spalte bzw. Zeile der Tabelle 70 ermittelt wurde, kann in der Tabelle 70 der jeweilige Konzentrationswert abgelesen (hier 20%) oder anhand der Position der jeweiligen Zeile ermittelt werden (zum Beispiel: n-te Zeile entspricht n*x%). Die Tabelle 70 ist damit eine Tabelle nach Art einer grundsätzlich an sich bekannten Umsetzungstabelle (Look-Up-Tabelle) 70, so dass für noch weitergehende Details zum Zugriff auf die Daten der Tabelle 70 auf bekannte Beschreibungen in der Fachliteratur verwiesen werden kann.

Selbstverständlich ist mittels der Tabelle 70 optional auch eine Interpolation von Zwischenwerten möglich, zum Beispiel derart, dass anhand eines Temperaturmesswerts eine der Spalten der Tabelle 70 ausgewählt wird und bei einem zwischen zwei benachbarten Daten dieser ausgewählten Spalte liegenden Messergebnis der tatsächliche Konzentrationswert auf Basis einer Interpolation der zu diesen gehörenden Konzentrationswerte gemäß der Tabelle 70 gebildet wird.

Wenn anhand der Tabelle 70 ein Konzentrationswert oberhalb eines vorgegebenen oder vorgebbaren Schwellwerts ermittelt wird, erzeugt der Glykolsensor 10 ein Sensorsignal 26 zur Ansteuerung eines Ventils 18 oder eines sonstigen Aktors, so dass ein Ausfluss des Wasser-Glykol-Gemisches 22 aus der Auffangwanne 14 verhindert wird. Bei einer Verwendung des Glykolsensors 10 in einem anderen Szenario (Dachentwässerung, Rohrsystem oder dergleichen) erfolgt eine Ansteuerung eines dort vorgesehenen Aktors, zum Beispiel ebenfalls eine Ansteuerung eines Ventils oder einer Gruppe von Ventilen.

Optional umfasst der Glykolsensor 10 einen Drucksensor (nicht gezeigt) oder dem Glykolsensor 10 ist ein Drucksensor zugeordnet. Mittels des Drucksensors ist im Betrieb ein Drucksensor-Sensorsignal als Maß für einen Druck generierbar, dem der Glykolsensor 10 ausgesetzt ist - zum Beispiel in einer Sensoraufnahmevorrichtung 50 aufgrund von unter Druck durch die Sensoraufnahmevorrichtung 50 strömendem Fluid oder in einem Rohr aufgrund von unter Druck durch das Rohr strömendem Fluid. Mittels eines solchen Drucksensor-Sensorsignals ist optional eine Druckkompensation des vom Glykolsensor 10 im Betrieb generierten Sensorsignals 26 möglich. Die Druckkompensation kann dabei zum Beispiel ähnlich realisiert sein, wie dies zuvor für die Temperaturkompensation beschrieben wurde. D.h. es wird zum Beispiel eine weitere Tabelle verwendet, in welcher für unterschiedliche Druckwerte jeweils ein Gewichtsfaktor gespeichert ist (optionale Interpolation bei Zwischenwerten). Der Schallgeschwindigkeits-Messwert des Glykolsensors 10 wird mit diesem Gewichtsfaktor multipliziert (gewichtet) und mit dem resultierenden Wert erfolgt zum Beispiel der Zugriff auf die Tabelle 70 zum Erhalt des jeweiligen Konzentrationswerts.

### Bezugszeichenliste

- 10: Ultraschall- / Glykolsensor
- 12: Flüssigkeitsrückhaltesystem
- 14: Auffangwanne
- 16: Abscheider / Flüssigkeitsrückhaltevorrichtung
- 18: Ventil
- 20: Aggregat
- 22: Wasser-Glykol-Gemisch
- 24: Abfluss
- 26: Sensorsignal
- 28: (frei)
- 30: Gehäuse
- 32: Halterung
- 34: Sender
- 36: Empfänger
- 38: Temperatursensor
- 40: Steuerungseinheit
- 42: Schutzschicht
- 44: Schutzschicht
- 46: Schutzschicht
- 48: Anschlusskabel
- 50: Sensoraufnahmevorrichtung
- 52: Bodenabschnitt (an der Sensoraufnahmevorrichtung)
- 54: Einstülpung (in der Sensoraufnahmevorrichtung und zur Aufnahme des Ultraschall- / Glykolsensors)
- 56, 58: (frei)
- 60: Steuerungsprogramm
- 62: Startsignal
- 64: Ultraschallsignal
- 66: Empfangssignal
- 68: Temperatursignal
- 70: Umsetzungstabelle

## Patentansprüche

1. Flüssigkeitsrückhaltesystem (12) mit zumindest einer unter einem Aggregat (20) einer leckagegefährdeten Anlage platzierten Auffangwanne (14) und einem Ultraschallsensor (10),
wobei mittels des Ultraschallsensors (10) ein Sensorsignal (26) generierbar ist, welches eine Erkennung von Glykol in einem Wasser-Glykol-Gemisch (22), dem der Ultraschallsensor (10) ausgesetzt ist, kodiert und
wobei mittelbar oder unmittelbar aufgrund des Sensorsignals (26) ein stromabwärts der Auffangwanne (14) platziertes Ventil (18) ansteuerbar ist,
**dadurch gekennzeichnet,**
**dass** der Ultraschallsensor (10) einen Sender (34) und einen Empfänger (36) zum Aussenden bzw. Empfangen von Ultraschallsignalen (64) sowie eine Steuerungseinheit (40) umfasst,
**dass** mittels der Steuerungseinheit (40) ein Maß für eine Laufzeit eines UItraschallsignals (64) vom Sender (34) zum Empfänger (36) ermittelbar ist und
**dass** mittels der Steuerungseinheit (40) anhand des ermittelten Maßes für die Laufzeit des Ultraschallsignals (64) und anhand eines Temperaturmesswerts eine Glykolkonzentration in einem Wasser-Glykol-Gemisch (22) ermittelbar ist, dem der Ultraschallsensor (10) ausgesetzt ist.

2. Flüssigkeitsrückhaltesystem (12) nach Anspruch 1,
wobei der Ultraschallsensor (10) einen Temperatursensor (38) umfasst und mittels der Steuerungseinheit (40) anhand eines vom Temperatursensor (38) erhältlichen Temperatursignals (68) ein Temperaturmesswert ermittelbar ist.

3. Flüssigkeitsrückhaltesystem (12) nach Anspruch 1 oder Anspruch 2,
wobei die Glykolkonzentration in dem Wasser-Glykol-Gemisch (22), dem der Ultraschallsensor (10) ausgesetzt ist, mittels der Steuerungseinheit (40) mittels einer Umsetzungstabelle (70) und darin gespeicherter, empirisch ermittelter Werte ermittelbar ist,
wobei die Umsetzungstabelle (70) Daten für verschiedene Temperaturen und verschiedene Glykolkonzentrationen umfasst und
wobei der Zugriff auf die Umsetzungstabelle (70) mittels des Temperaturmesswerts und anschließend mittels des ermittelten Maßes für die Laufzeit des Ultraschallsignals (64) erfolgt.

4. Flüssigkeitsrückhaltesystem (12) nach einem der Ansprüche 1, 2 oder 3, mit einem Ultraschallsensor (10) mit einer Busschnittstelle.

5. Flüssigkeitsrückhaltesystem (12) nach einem der vorangehenden Ansprüche,
wobei der Ultraschallsensor (10) stromabwärts der Auffangwanne (14) in einer Sensoraufnahmevorrichtung (50) platziert ist,
wobei die Sensoraufnahmevorrichtung (50) einen in Relation zu einem Bodenniveau der Auffangwanne (14) tiefer liegenden Bodenabschnitt (52) umfasst, in welchem sich zumindest ein Teil eines aus der Auffangwanne (14) abfließenden Fluids sammelt,
wobei der Ultraschallsensor (10) an einer Halterung (32) einen Ultraschall-Sender (34) und einen Ultraschall-Empfänger (36) aufweist und
wobei der Ultraschallsensor (10) von der Sensoraufnahmevorrichtung (50) in einer Art und Weise aufgenommen ist, dass Ultraschall-Sender (34) und Ultraschall-Empfänger (36) in den Bereich des tiefer liegenden Bodenabschnitts (52) reichen.

6. Flüssigkeitsrückhaltesystem (12) nach Anspruch 5,
wobei die Sensoraufnahmevorrichtung (50) eine von einer Hüllfläche der Sensoraufnahmevorrichtung (50) zurückgesetzte Einstülpung (54) umfasst,
wobei der Ultraschallsensor (10) in der Sensoraufnahmevorrichtung (50) am Boden der Einstülpung (54) fixiert ist,
wobei eine zumindest den Ultraschall-Sender (34) und den Ultraschall-Empfänger (36) umfassende Sensorik des Ultraschallsensors (10) im Innern der Sensoraufnahmevorrichtung (50) an eine Innenseite des Bodens der Einstülpung (54) anschließt und
wobei eine Steuerungseinheit (40) des Ultraschallsensors (10) sich auf einer der Innenseite des Bodens der Einstülpung (54) gegenüberliegenden Seite befindet.

7. Flüssigkeitsrückhaltesystem (12) nach Anspruch 6,
wobei die Sensorik (34, 36, 38) des Ultraschallsensors (10) sich unmittelbar im Anschluss an den Boden der Einstülpung (54) im Innern der Sensoraufnahmevorrichtung (50) befindet und die Steuerungseinheit (40) sich ebenfalls unmittelbar im Anschluss an den Boden der Einstülpung (54) auf der gegenüberliegenden Seite des Bodens der Einstülpung (54) befindet.

8. Flüssigkeitsrückhaltesystem (12) nach einem der vorangehenden Ansprüche,
wobei der Ultraschallsensor (10) als Sensorsignal (26) ein Binärsignal erzeugt,
welches mit einem ersten Status kodiert, dass kein Glykol oder nur eine Glykolkonzentration unterhalb eines vorgegebenen oder vorgebbaren Schwellwerts sensiert wurde, und mit einem komplementären zweiter Status kodiert, dass Glykol oder eine Glykolkonzentration oberhalb des Schwellwerts sensiert wurde.

9. Flüssigkeitsrückhaltesystem (12) nach einem der vorangehenden Ansprüche, mit einem aufgrund eines Sensorsignals (26) vom Ultraschallsensor (10) ansteuerbaren Ventil (18) stromabwärts der Auffangwanne (14) und stromabwärts der Sensoraufnahmevorrichtung (50).

10. Verfahren zum Betrieb eines Flüssigkeitsrückhaltesystems (12) nach einem der vorangehenden Ansprüche, wobei mittels des Ultraschallsensors (10) ein Sensorsignal (26) generiert wird, welches eine Erkennung von Glykol in einem Wasser-Glykol-Gemisch (22), dem der Ultraschallsensor (10) ausgesetzt ist, kodiert und wobei mittelbar oder unmittelbar aufgrund des Sensorsignals (26) das Ventil (18) angesteuert wird.
